(19) Europäisches Patentamt
European Patent Office
Office européen des brevets

(11) Publication number: **0 420 266 A2**

# (12) EUROPEAN PATENT APPLICATION

(21) Application number: 90118678.3

(22) Date of filing: 28.09.90

(51) Int. Cl.[5]: C07D 311/58, C07D 335/06, C07D 311/96, C07D 407/06, C07D 409/06, A61K 31/35, A61K 31/38

(30) Priority: 29.09.89 JP 256226/89

(43) Date of publication of application: 03.04.91 Bulletin 91/14

(84) Designated Contracting States: AT BE CH DE DK ES FR GB GR IT LI LU NL SE

(71) Applicant: TANABE SEIYAKU CO., LTD.
2-10, Dosho-machi 3-chome
Chuo-ku Osaka(JP)

(72) Inventor: Nakai, Hideo
No. 19-18, Hanyashiki-matsugaoka
Takarazuka-shi, Hyogo-ken(JP)
Inventor: Yamada, Koichiro
No. 34-7, Shinshiraoka 3-chome,
Shiraoka-machi
Minamisaitama-gun, Saitama-ken(JP)
Inventor: Nomura, Sumihiro
No. 4-11-509, Hikonari 4-chome
Misato-shi, Saitama-ken(JP)
Inventor: Takashima, Kohki
No. 1-3-904, Higashi-kasai 5-chome
Edogawa-ku Tokyo-to(JP)
Inventor: Suzuki, Kazuko
304 Fujiwara Mansion 46-25 Kitazono-cho
Kawaguchi-shi Saitama-ken(JP)

(74) Representative: Hansen, Bernd, Dr.
Dipl.-Chem. et al
Hoffmann, Eitle & Partner Patent- und
Rechtsanwälte Arabellastrasse 4
W-8000 München 81(DE)

## (54) Chromene or thiochromene derivatives, process for preparing the same, intermediate therefor, pharmaceutical compositions containing said derivatives and the use of said derivatives.

(57) A chromene or thiochromene derivative of the formula:

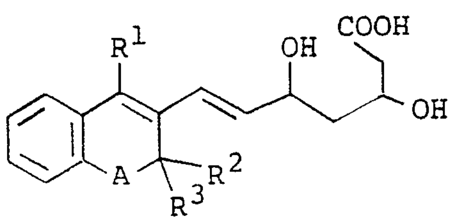

(I)

wherein A is oxygen or sulfur atom, $R^1$ is a halogenophenyl, $R^2$ and $R^3$ are the same or different and are each a lower alkyl or $R^2$ and $R^3$ are combined together to form $-(CH_2)_n-$ in which n is an integer of 4 to 6, or a pharmaceutically acceptable ester, amide, lactone or salt thereof, which has excellent HMG-CoA reductase inhibitory activity and is useful for prophylaxis and treatment of hyperlipidemic diseases, a process for preparing said compound and an intermediate compound used for preparing said compound. Further, a pharmaceutical composition containing the above mentioned chromene or thiochromene derivatives is disclosed.

EP 0 420 266 A2

# CHROMENE OR THIOCHROMENE DERIVATIVES, PROCESS FOR PREPARING THE SAME, INTERMEDIATE THEREFOR, PHARMACEUTICAL COMPOSITIONS CONTAINING SAID DERIVATIVES AND THE USE OF SAID DERIVATIVES

The present invention relates to new chromene or thiochromene derivatives which are useful as an antihyperlipidemic agent. The compounds of the present invention exhibit anti-hyperlipidemic activity through inhibition of HMG-CoA reductase.

## Prior Art

Hyperlipidemia is considered to be one of main factors causing arteriosclerosis which is one of adult diseases. There have been known various agents for treatment and prophylaxis of hyperlipidemia including clofibrate [chemical nomenclature: 2-(4-chlorophenoxy)-2-methylpropanoic acid ethyl ester], probucol [chemical nomenclature: 4,4′-[(1-methylethylidene)bis(thio)]bis[2,6-bis(1,1-dimethylethyl)phenol]] and the like, which exhibit the anti-hyperlipidemic activity through inhibition of absorption of cholesterol and bile acid, or inhibition of synthesis and secretion of very low density lipoprotein (hereinafter referred to as "VLDL").

On the other hand, it is known that 3-hydroxy-3-methylglutaryl coenzyme A reductase (hereinafter referred to as "HMG-CoA reductase") catalyzes the conversion of 3-hydroxy-3-methylglutaryl coenzyme A (hereinafter referred to as "HMG-CoA") to mevalonic acid. Therefore, cholesterol biosynthesis can be inhibited by inhibiting said enzyme. At present, there is a demand to develop a novel anti-hyper lipidemic agent which can exhibit the anti-hyperlipidemic activity based on the above-mentioned mechanism different from the conventional agent.

## Brief Description of the Invention

An object of the present invention is to provide a novel compound which is useful as an anti-hyperlipidemic agent based on HMG-CoA reductase inhibiting activity.

Another object of the invention is to provide a process for preparing said novel compcund.

A further object of the invention is to provide a pharmaceutical composition for the prophylaxis and treatment of hyperlipidemia comprising said novel compound as an active ingredient.

A still further object of the invention is to provide a novel intermediate useful for preparing said useful compound.

These and other objects and advantages of the invention are apparent to those skilled in the art from the following description.

## Detailed Description of the Invention

The novel compound of the present invention is a chromene or thiochromene derivative of the formula:

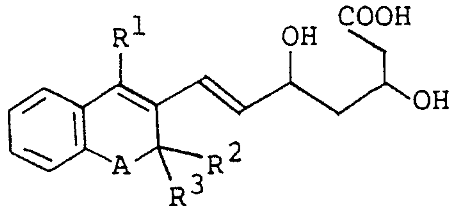

(I)

wherein A is oxygen or sulfur atom, $R^1$ is a halogenophenyl, $R^2$ and $R^3$ are the same or different and are each a lower alkyl or $R^2$ and $R^3$ are combined together to form $(CH_2)_n$- in which n is an integer of 4 to 6, or a pharmaceutically acceptable ester, amide, lactone or salt thereof.

The compound (I) of the present invention has an excellent HMG-CoA reductase inhibiting activity, and

hence, is useful as antihyperlipidemic agents, especially as agents for prophylaxis and treatment of hypercholesterolemia.

The compounds of the present invention include the compound (I) wherein $R^1$ is a phenyl group which is substituted by one or more of a halogen atom, $R^2$ and $R^3$ are the same or different and are each an alkyl group having 1 to 4 carbon atoms, or $R^2$ and $R^3$ are combined together to form $-(CH_2)_n-$ in which n is an integer of 4, 5 or 6.

An ester of the compound (I) of the present invention includes a lower alkyl ester, a phenyl(lower)alkyl ester, and the like. An amide of the compound (I) of the present invention includes a non-substituted amide, a mono(lower alkyl)amide, di(lower alkyl)amide, and the like. A lactone of the compound (I) of the present invention includes the compound of the formula:

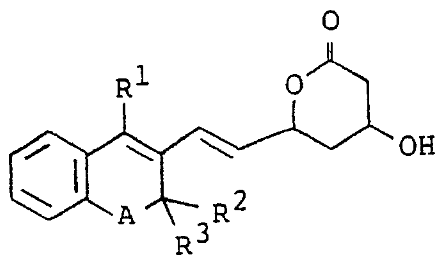

(I-a)

wherein A, $R^1$, $R^2$ and $R^3$ are as defined above.

Among the compounds (I) of the present invention, preferred one in view of treatment of the disease is the compound (I) wherein $R^2$ and $R^3$ are ethyl. Another preferred one in view of treatment of the disease is the compound (I) wherein A is oxygen atom and $R^2$ and $R^3$ are the same or different and are each an alkyl group having 1 to 4 carbon atoms, or wherein A is sulfur atom and $R^2$ and $R^3$ are ethyl. In the formula (I), the lower alkyl group for $R^2$ and $R^3$ is preferably an alkyl group having 1 to 4 carbon atoms and the halogenophenyl group for $R^1$ is preferably fluorophenyl.

The compounds (I) of the present invention cover both optical isomers thereof and a mixture of these isomers, among which (3R, 5S)-isomer is preferred in case of the non-lactone type and (4R, 6S)-isomer is preferred in case of the lactone type.

The compound (I) of the present invention can be prepared, for example, by reacting an aldehyde compound of the formula:

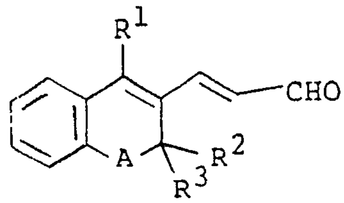

(II)

wherein A, $R^1$, $R^2$ and $R^3$ are as defined above, with an acetoacetic acid compound of the formula:

$CH_3COCH_2COOR^4$ (III)

wherein $-COOR^4$ is a carboxyl group which may have a protecting group, or a salt thereof, reducing the resulting intermediate compound of the formula:

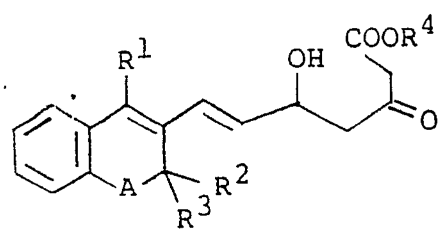

(IV)

wherein A, $R^1$, $R^2$, $R^3$ and $-COOR^4$ are as defined above, or a salt thereof, and removing optionally the protecting group when $-COOR^4$ is a carboxyl group having a protecting group.

The protecting group in the starting acetoacetic acid compound (III) and the intermediate compound (IV) may be any protecting group which can easily be removed by a usual procedure such as hydrolysis, reduction, solvolysis, acid treatment and the like, and includes a lower alkyl group, a lower alkyl group substituted by a substituted or non-substituted phenyl group (e.g. benzyl, p-methoxybenzyl, p-nitrobenzyl, etc.), benzhydryl group and the like.

The reaction between the aldehyde compound (II) and the acetoacetic acid compound (III) or a salt thereof is preferably carried out in the presence of a base. The base includes, for example, an alkali metal hydride (e.g. sodium hydride, potassium hydride, etc.), a (lower alkyl) lithium (e.g. n-butyllithium, etc.), diisopropylamide, and the like. The acetoacetic acid compound (III) in which $-COOR^4$ is a free carboxyl group can also be used in the reaction in the form of an alkali metal salt. The reaction is preferably conducted at -78 °C to 30 °C, preferably at -10 °C to 5 °C.

The reduction of the intermediate compound (IV) can be carried out using a reducing agent capable of selectively reducing the ketone moiety of the compound (IV) (e.g. an alkali metal borohydride such sodium borohydride) or a combination of the reducing agent with a boron compound such as tri(lower alkyl)boron (e.g. triethylboron, tri(n-butyl)boron, etc.) or di(lower alkyl)alkoxyboron (e.g. diethylmethoxyboron, etc.). When the intermediate compound (IV) is a free carboxylic acid, it may be used in the reaction as an alkali metal salt thereof. The reaction is preferably carried out in the presence of a suitable solvent (e.g. ether, tetrahydrofuran, dioxane, or a mixture thereof) under cooling condition or at room temperature.

The compound (I) in the form of an ester prepared in the above reduction reaction can optionally be converted into the corresponding free carboxylic acid by removing the protecting group ($R^4$). This conversion can be carried out by a usual procedure such as hydrolysis, reduction, solvolysis, acid treatment and the like. For example, the compound (I) in the form of an ester is hydrolyzed with a base in a solvent such as water, a lower alkanol, or a mixture of water and an organic solvent (e.g. tetrahydrofuran, dioxane, etc.) and neutralizing the product with an acid to give the desired compound (I) in the form of a free carboxylic acid. The base includes an alkali metal hydroxide, an alkali metal carbonate, an alkali metal hydrogen carbonate, and the like. The acid for neutralization includes a mineral acid such as hydrochloric acid. The reaction can preferably be carried out at room temperature to 50 °C, preferably at room temperature.

The amide and lactone of the compound (I) of the present invention can be prepared from the compound (I) in the form of a free carboxylic acid as prepared above by a usual procedure. For example, the amide can be prepared by reacting the compound (I) in the form of a free carboxylic acid with an amine compound (e.g. ammonia, a lower alkylamine, di(lower alkyl)amine, etc.). This reaction can be carried out in the amine compound used or in a suitable solvent such as benzene or toluene. The compound (I-a) in the form of a lactone can be prepared by subjecting the compound (I) in the form of a free carboxylic acid to an intramolecular esterification reaction. The reaction is preferably conducted in a solvent such as benzene, toluene, xylene under reflux temperature. The ester of the compound (I) of the present invention can also be prepared by reacting the compound (I) in the form of a free carboxylic acid with a lower alkanol or a phenyl-(lower)alkanol in the presence of an acid catalyst (e.g. hydrogen chloride, sulfuric acid, p-toluenesulfonic acid, strong acid ion exchange resin, etc.).

The starting compound (II) of the present invention is a novel compound and can be prepared, for example, by reacting a compound of the formula:

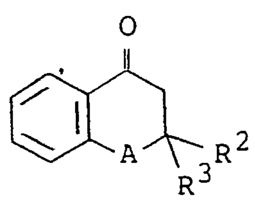

(V)

wherein A, $R^2$ and $R^3$ are as defined above, which is prepared in accordance with a process disclosed in Journal of Chemical Society, 2010 (1956), with a compound of the formula:

$R^1X$ (VI)

wherein $R^1$ is as defined above and X is a reactive residue, in the presence of n-butyllithium, dehydrating the reaction product, brominating at 3-position of the chromene or thiochromene ring, followed by reacting the product with ethyl acrylate in accordance with a process disclosed in Journal of Organic Chemistry, 40, 1083 (1975) to give the compound of the formula:

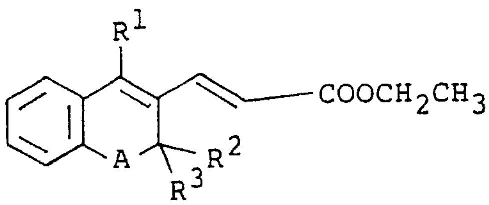

(VII)

wherein A, $R^1$, $R^2$ and $R^3$ are as defined above, and reducing the compound so that the ethoxycarbonyl group is converted into hydroxyl group, and finally oxidizing the product with an oxidizing agent such as manganese dioxide.

The compound (II) of the present invention wherein A is sulfur atom [compound (IIa)] can also be prepared by

(1) reacting thiophenol and a carboxylic acid of the formula:

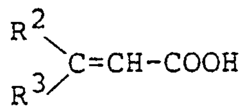

(VIII)

wherein $R^2$ and $R^3$ are the same as defined above, under either acidic or basic condition to give a compound of the formula:

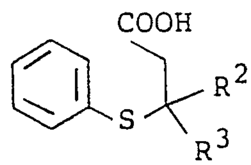

(IX)

wherein $R^2$ and $R^3$ are the same as defined above,

(2) cyclizing the compound (IX) either by treatment with polyphosphoric acid or by subjecting the acid halide of the compound (IX) to Friedel-Crafts reaction,

(3) reacting the cyclized product with the compound (VI) in the presence of n-butyllithium, dehydrating the reaction product to give a compound of the formula:

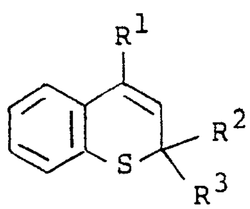

(X)

wherein $R^1$, $R^2$ and $R^3$ are the same as defined above,

(4) brominating the compound (X) at 3-position thereof, and

(5) reacting the brominated compound with a trialkylstannyl-2-propen-1-ol, followed by oxidizing the product with an oxidizing agent such as manganese dioxide.

The compounds (I) of the present invention, and a pharmaceutically acceptable ester, an amide, a lactone and a salt thereof, have an excellent activity to inhibit HMG-CoA reductase, and hence are useful as anti-hyperlipidemic agents and can be used for treatment, relief and prophylaxis of hyperlipidemia, coronary disease, arteriosclerosis, familial hypercholesterolemia and xanthoma, and other related disease.

The pharmaceutically acceptable salt of the compounds (I) of the present invention includes an alkali metal salt (e.g. sodium salt, potassium salt, etc.), an alkaline earth metal salt (e.g. calcium salt, magnesium salt, etc.), a heavy metal salt (e.g. zinc salt, etc.), and a salt with an organic amine (e.g. ammonium salt, triethylamine salt, pyridine salt, ethanolamine salt, basic amino acid salts, etc.).

The compounds (I) of the present invention, and its ester, amide, lactone and salt, can be administered

both via oral route and parenteral route (e.g. intravenously, intramuscularly, intraperitoneally, topically etc.) by formulating into conventional pharmaceutical preparations in a suitable dosage form including tablets, granules, capsules, powders, injections and the like by a usual procedure.

The dosage of the compounds of the present invention may vary depending on an administration route, age, weight and conditions of the patients, but it is usually in the range of about 0.05 to about 10 mg/kg per day, preferably about 0.1 to about 5 mg/kg per day.

The compounds (I) of the present invention were tested for their activity to inhibit HMG-CoA reductase (HMGR).

Experiment [Inhibitory Activity against Rat Hepatic Microsomal HMG-CoA reductase (HMGR)]

HMGR activities of microsomes were measured in accordance with the method of N. L. Young et al., Methods in Enzymology, 71 , 498 (1981).

Microsomes prepared from liver of rats administered with cholestyramine were mixed with a solution of the compounds to be tested in dimethyl sulfoxide and $^{14}$C-HMG-CoA (substrate). After the mixture was incubated for 10 minutes, the reaction was quenched with 6N hydrochloric acid and the mixture was allowed to stand at 37° C for 15 minutes, followed by centrifugation. The supernatant was spotted on thin layer chromatography (TLC; Kieselgel 60F254 manufactured by Merck) and developed with toluene/acetone (1:1). The spot of $^{14}$C-mevalonolactone was collected and the amount of isotope was measured with a scintillation counter (Type 4640 TRI-CARB. manufactured by Paccard) to calculate an amount of the formed mevalonolactone. By comparing amounts of the formed mevalonolactone in the group treated with test compounds and the control group (without test compounds), HMGR activity inhibition rates of the test compounds were calculated. The results are shown in Table 1.

Table 1

| HMGR Inhibition (%) | | |
|---|---|---|
| Test Compounds | Conc. of Compounds (M) | |
| | $10^{-6}$ | $10^{-7}$ |
| A | 100 | 67 |
| B | 84 | 32 |
| C | 90 | 45 |
| Test compounds: | | |
| A: (3R*,5S*)-(E)-7-[4-(4-Fluorophenyl)-2,2-diethyl-2H-chromen-3-yl]-3,5-dihydroxy-6-heptenoic acid sodium salt | | |
| B: Trans-(E)-6-[2-(4-(4-fluorophenyl)-2,2-diethyl-2H-chromen-3-yl-)-1-ethenyl]-3,4,5,6-tetrahydro-4-hydroxy-2H-pyran-2-one | | |
| C: (+)-(3R*,5S*)-(E)-7-[4-(4-Fluorophenyl)-2,2-diethyl-2H-thiochromen-3-yl]-3,5-dihydroxy-6-heptenoic acid sodium salt | | |

The present invention is explained in more detail by the following Examples and Reference Examples

but should not be construed to be limited thereto.

Example 1

(E)-3-[4-( 4-Fluorophenyl)-2,2-dimethyl-2H-chromen-3-yl]-2-propenal:

A. 3-Bromo-4-(4-fluorophenyl)-2,2-dimethyl-2H-chromene:

To an ice-cooled solution of 4-(4-fluorophenyl)-2,2-dimethyl-2H-chromene (0.567 g) in methylene chloride (22 ml) was added pyridinium bromide perbromide (0.87 g) and the mixture was stirred at room temperature for 1 hour. The solution was washed, dried, and evaporated in vacuo. The residue was recrystallized from petroleum ether to give the title compound (0.63 g) as colorless needles.
Yield: 85 %
M.P.: 121 - 122 °C

B. Ethyl (E)-3-[4-(4-fluorophenyl)-2,2-dimethyl-2H-chromen-3-yl]-2-propenoate:

A reaction vessel was charged with a mixture of the compound A (2.20 g), ethyl acrylate (13.2 g), palladium acetate (296 mg), triphenylphosphine (692 mg) and triethyl amine (13.35 g) and purged with argon and then sealed. After heating at 160 to 170 °C for 24 hours, the reaction mixture was diluted with ethyl acetate and the insoluble materials were removed by filtration. The filtrate was evaporated and the residue was purified by silica-gel flash column chromatography [eluent: n-hexane/ethyl acetate (20:1)] and recrystallized from n-hexane to give the title compound (1.62 g) as pale yellow needles.
Yield: 70 %
M.P.: 113.5 - 114.0 °C

C. (E)-3-[4-(4-fluorophenyl)-2,2-dimethyl-2H-chromen-3-yl]-2-propen-1-ol:

To a mixture of the compound B (1.37 g) and dry methylene chloride (14 ml) was dropwise added a solution of diisobutyl aluminium hydride in toluene (1.5 M, 7.8 ml) over 5 minutes in an ice-ethanol bath. After stirring at -5 to 0 °C for 50 minutes, 10% hydrochloric acid was added and the mixture was extracted with ethyl acetate. The extract was washed, dried and evaporated in vacuo to give the title compound (1.2 g) as oil.
Yield: 100 %
IR $\nu$ ($CHCl_3$) : 3600, 1600 $cm^{-1}$
MS (m/z): 310 ($M^+$), 295

D. (E)-3-[4-(4-Fluorophenyl)-2,2-dimethyl-2H-chromen-3-yl]-2-propenal:

A mixture of the compound C (1.2 g) and manganese dioxide (3.4 g) in dry methylene chloride (30 ml) was refluxed for 1 hour. After cooling, the insoluble materials were removed by filtration and washed with chloroform. The washings and the filtrate were combined together and evaporated in vacuo. The residue was recrystallized from ethyl acetate/n-hexane to give the title compound (1.01 g) as yellow needles.
Yield: 84 %
M.P.: 158.5 - 159.5 °C
IR $\nu$ (Nujol): 1670, 1610, 1600, 1590 $cm^{-1}$
MS (m/z): 308 ($M^+$), 293

Example 2

(E)-3-[4-(4-Fluorophenyl)-2,2-diethyl-2H-chromen-3-yl]-2-propenal:

A. 3-Bromo-4-(4-fluorophenyl)-2,2-diethyl-2H-chromene:

The procedure in Example 1(A) was repeated using 4-(4-fluorophenyl)-2,2-diethyl-2H-chromene in place of 4-(4-fluorophenyl)-2,2-dimethyl-2H-chromene to give the title compound.

B. Ethyl (E)-3-[4-(4-fluorophenyl)-2,2-diethyl-2H-chromen-3-yl]-2-propenoate:

The compound A was treated in the same manner as described in Example 1(B) to give the title compound as yellow oil.
IR ν (neat): 1700, 1610, 1590 $cm^{-1}$
MS (m/z) : 380($M^+$), 351

C. (E)-3-[4-(4-fluorophenyl)-2,2-diethyl-2H-chromen-3-yl]-2-propen-1-ol:

The compound B was treated in the same manner as described in Example 1(C) to give the title compound as colorless crystals.
M.P.: 87 - 90 °C
IR ν (Nujol): 3300, 1605 $cm^{-1}$
MS (m/z): 338 ($M^+$), 309

D. (E)-3-[4-(4-fluorophenyl)-2,2-diethyl-2H-chromen-3-yl]-2-propenal:

The compound C was treated in the same manner as described in Example 1(D) to give the title compound as yellow prisms.
M.P.: 126 - 128 °C (recrystallized from petroleum ether)
IR ν (Nujol): 1675, 1590 $cm^{-1}$
MS (m/z): 336 ($M^+$), 307

Example 3

Methyl (3R*,5S*)-(E)-7-[4-(4-fluorophenyl)-2,2-dimethyl-2H-chromen-3-yl]-3,5-dihydroxy-6-heptenoate:

A. Methyl (E)-7-[4-(4-fluorophenyl)-2,2-dimethyl-2H-chromen-3-y1]-5-hydroxy-3-oxo-6-heptenoate:

A solution of methyl acetoacetate (745 mg) in tetrahydrofuran (1 ml) was dropwise added to an ice-cooled solution of 60 % sodium hydride (257 mg) in tetrahydrofuran (5 ml) and the mixture was stirred for 5 minutes. Thereto was dropwise added a solution of n-butyllithium in hexane (1.6 M, 4.0 ml) at -5 to 5 °C and the mixture was stirred for 15 minutes. To the mixture was dropwise added a solution of (E)-3-[4(4-fluorophenyl)-2, 2-dimethyl-2H-chromen-3-yl ]-2-propenal (0.99 g) in tetrahydrofuran (13 ml) over 10 minutes and the mixture was stirred for 50 minutes. After reaction was completed, an aqueous solution of ammonium chloride was added and the mixture was extracted with ethyl acetate. The extract was washed, dried and evaporated in vacuo. The residue was purified by silica-gel flash column chromatography [eluent: n-hexane/ethyl acetate (3:1)] to give the title compound (1.34 g) as pale yellow oil.
Yield: 98 %
IR ν ($CHCl_3$): 3600, 1745, 1720, 1655, 1600 $cm^{-1}$
MS (m/z): 424 ($M^+$), 409

B. Methyl (3R*,5S*)-(E)-7-[4-(4-fluorophenyl)-2,2-dimethyl-2H-chromen-3-yl]-3,5-dihydroxy-6-heptenoate:

To a cooled solution of the compound A (1.3 g) in tetrahydrofuran (13 ml) was dropwise added a solution of triethylborane in tetrahydropyran (1.0 M, 4.6 ml) under argon atmosphere and then 0.8 ml of air was blown into the mixture. After stirring at room temperature for 15 minutes, sodium borohydride (0.23 g) was added to the mixture in a dry ice-acetone bath. Then, methanol (3.0 ml) was added over 20 minutes and the mixture was stirred for another 30 minutes. The reaction mixture was poured into a mixture of ice and 30% hydrogen peroxide (7.3 ml), and extracted with ethyl acetate. The extract was washed, dried and evaporated in vacuo. The residue was purified by silica-gel flash column chromatography [eluent: n-hexane/ethyl acetate (1:1)] and recrystallized from n-hexane/ethylacetate to give the title compound (0.95 g) as colorless prisms.
Yield: 73 %
M.P.: 103 - 104 °C
IR $\nu$ (Nujol): 3400, 1720, 1600 $cm^{-1}$
MS (m/z): 426 ($M^+$), 411

Example 4

Methyl (3R*,5S*)-(E)-7-[4-(4-fluorophenyl)-2,2-diethyl-2H-chromen-3-yl]-3,5-dihydroxy-6-heptenoate:

A. Methyl (E)-7-[4-(4-fluorophenyl)-2,2-diethyl-2H-chromen-3-yl]-5-hydroxy-3-oxo-6-heptenoate:

The procedure described in Example 3(A) was repeated using (E)-3-[4-(4-fluorophenyl)-2,2-diethyl-2H-chromen-3-yl]-2-propenal in place of (E)-3-[4-(4-fluorophenyl)-2,2-dimethyl-2H-chromen-3yl]-2-propenal to give the title compound as pale orange oil.
IR $\nu$ (Neat): 3500, 1745, 1715, 1600 $cm^{-1}$
MS (m/z): 452 ($M^+$), 423

B. Methyl (3R*,5S*)-(E)-7-[4-(4-fluorophenyl)-2,2-diethyl-2H-chromen-3-yl]-3,5-dihydroxy-6-heptenoate:

The compound A was treated in the same manner as described in Example 3(B) to give the title compound as pale yellow oil.
111111 IR $\nu$ (Neat): 3420, 1730, 1600 $cm^{-1}$
MS (m/z): 454 ($M^+$), 425

Example 5

(3R*,5S*)-(E)-7-[4-(4-Fluorophenyl)-2,2-dimethyl 2H-chromen-3-yl]-3,5-dihydroxy-6-heptenoic acid:

To a stirred and ice-cooled solution of methyl (3R*,5S*)-(E)-7-[4-(4-fluorophenyl)-2,2-dimethyl-2H-chromen-3-yl]-3,5-dihydroxy-6-heptenoate (0.85 g) in methanol (6 ml) was dropwise added 1N aqueous sodium hydroxide (2.2 ml). After 30 minutes, the solvent was removed by distillation. The residue was then purified by a column filled with nonionic absorption resin (trade name: Daiyaion HP-20 manufactured by Mitsubishi Kasei K.K.) using an eluent [water, then water/methanol (1:1)] to give powder (0.64 g). A solution of the powder (0.30 g) in water (6 ml) was made acidic with 10% hydrochloric acid (1 ml) and extracted with ethyl acetate. The extract was washed, dried and evaporated in vacuo to give the title compound (0.28 g) as colorless oil.
IR $\nu$ ($CHCl_3$) : 3560 - 3280, 1730, 1600 $cm^{-1}$
MS (FAB) (m/z): 412 ($M^+$), 397
Sodium salt:
IR $\nu$ (Nujol): 3360, 1570, 1505, 1450 $cm^{-1}$
MS (FAB) (m/z): 457 ($M^+$ + Na), 435 ($M^+$ + 1), 397,

Example 6

(3R* ,5S*)-(E)-7-[4-(4-Fluorophenyl)-2,2-diethyl-2H-chromen-3-yl]-3,5-dihydroxy-6-heptenoic acid:

Methyl (3R*,5S*)-(E)-7-[4-(4-fluorophenyl)-2,2-diethyl-2H-chromen-3-yl]-3,5-dihydroxy-6-heptenoate was treated in the same manner as described in Example 5 to give the title compound as pale yellow oil.
IR ν ($CHCl_3$) : 3460, 1730, 1600 $cm^{-1}$
MS (FAB) (m/z): 463, 439 ($M^+$ - 1), 423
Sodium salt:
IR ν (KBr) : 3400, 1580 $cm^{-1}$
MS (FAB) (m/z): 485 ($M^+$ + Na), 463 ($M^+$ + 1), 115

Example 7

Trans-(E)-6-[2-(4-(4-fluorophenyl)-2,2-dimethyl-2H-chromen-3-yl)-1-ethenyl]-3,4,5,6-tetrahydro-4-hydroxy-2H-pyran-2-one:

A solution of (3R*,5S*)-(E)-7-[4-(4-fluorophenyl)-2,2-dimethyl-2H-chromen-3-yl]-3,5-dihydroxy-6-heptenoic acid (0.26 g) in toluene (20 ml) was refluxed with a Dean Stark apparatus for 5 hours. After evaporation of the solvent, the residue was dissolved in chloroform, washed and dried, and the chloroform was removed by distillation. The residue was recrystallized from ethyl acetate to give the title compound (0.17 g) as colorless needles.
Yield: 68 %
M.P.: 192 - 193 °C
IR ν (Nujol): 3340, 1705, 1655, 1600 $cm^{-1}$
MS (m/z): 394 ($M^+$), 379

Example 8

Trans-(E)-6-[2-(4-(4-Fluorophenyl)-2,2-diethyl-2H-chromen-3-yl)-1-ethenyl]-3,4,5,6-tetrahydro-4-hydroxy-2H-pyran-2-one:

(3R*,5S*)-(E)-7-[4-(4-fluorophenyl)-2,2-diethyl-2H-chromen-3yl-]-3,5-dihydroxy-6-heptenoic acid was treated in the same manner as described in Example 7 to give the title compound as crystals.
M.P.: 156 - 157 °C (recrystallized from ethyl acetate/isopropylether)
IR ν (Nujol): 3440, 1710, 1590 $cm^{-1}$
MS (m/z): 422 ($M^+$), 393

Example 9

Methyl (3R*,5S*)-(E)-7-[2,2-diethyl-4-(4-fluorophenyl)-2H-thiochromen-3yl]-3,5-dihydroxy-6-heptenoate:

A. Methyl (E)-7-[2,2-diethyl-4-(4-fluorophenyl)-2H-thiochromen-3-yl]-5-hydroxy-3-oxo-6-heptenoate:

A mixture of 60 % sodium hydride (0.73 g), tetrahydrofuran (10 ml) and methyl acetoacetate (2.12 g) was stirred under argon atmosphere at room temperature for 10 minutes. The reaction mixture was cooled at -5 to 0 °C and thereto was dropwise added a solution of n-butyllithium in hexane (1.6 N, 11.4 ml). After stirring at -5 °C for 15 minutes, there was dropwise added a solution of (E)-3-[-2,2- diethyl-4-(4-fluorophenyl)-2H-thiochromen-3-yl]-2-propenal (3.22 g) in tetrahydrofuran (22 ml) and the whole was stirred at -5 °C for 15 minutes. After the reaction was completed, ice and an aqueous saturated ammonium

chloride solution were added and the mixture was extracted with ethyl acetate. The extract was washed, dried and evaporated in vacuo. The residue was purified by silica-gel column chromatography [eluent: n-hexane/ethyl acetate (4:1)] to give the title compound (4.22 g) as yellow oil.

Yield: 99 %

MS (m/z): 468 ($M^+$), 439

IR (Liquid) $\nu_{Max}$: 3500, 1745, 1720 $cm^{-1}$

B. Methyl (3R*,5S*)-(E)-7-[2,2-diethyl-4-(4-fluorophenyl)-2H-thiochromen-3-yl]-3,5-dihydroxy-6-heptenoate:

To a solution of the compound obtained above (4.14 g) in tetrahydrofuran (24 ml) was added a solution of triethylborane in tetrahydrofuran (1 M, 9.7 ml) under argon atmosphere at room temperature. Then, 1 ml of air was blown into the mixture. After 10 minutes, the mixture was cooled to -70 °C, and there were added sodium borohydride (0.67 g) and methanol (8.6 ml). The whole mixture was stirred for 30 minutes. There was added a 30 % aqueous hydrogen peroxide solution in portions and the mixture was stirred at room temperature for 20 minutes. The mixture was diluted with water and extracted with ethyl acetate. The extract was washed, dried and evaporated in vacuo. The residue was purified by silica-gel column chromatography [eluent: n-hexane/ethyl acetate (1:1)] to give the title compound (3.37 g) as colorless oil.

Yield: 81 %

MS (m/z): 470 ($M^+$), 441

IR (Liquid) $\nu_{Max}$: 3440, 1740, 1505 $cm^{-1}$

Example 10

Sodium (3R*,5S*)-(E)-7-[2,2-diethyl-4-(4-fluorophenyl)-2H-thiochromen-3-yl]-3,5-dihydroxy-6-heptenoate:

A mixture of the compound prepared in Example 9(B) (3.35 g), methanol (30 ml) and 1N sodium hydroxide (28.5 ml) was stirred at room temperature for 1 hour. After completion of the reaction, the solvent was removed by distillation and the residue was purified by column chromatography [eluent: methanol/water (1:1)] using nonionic absorption resin (trade name: Daiyaion HP-20 manufactured by Mitusbishi Kasei K.K.) to give the title compound (2.51 g) as pale yellow powder.

Yield: 74 %

MS (FAB) (m/z): 501 ($M^+$ + Na), 479 ($M^+$ + 1), 177

IR (Nujol) $\nu_{Max}$: 3360, 1640, 1600, 1580, 1380 $cm^{-1}$

Example 11

(±)-Trans-(E)-6-[2-[-2,2-diethyl-4-(4-fluorophenyl)-2H-thiochromen-3-yl]-1-ethenyl]-3,4,5,6-tetrahydro-4-hydroxy-2H-pyran-2-one:

To a solution of the compound prepared in Example 10 (2.36 g) in water (30 ml) was added 1N hydrochloric acid (5 ml) and the mixture was extracted with chloroform. The extract was washed, dried, and evaporated in vacuo. The residue was taken into toluene (77 ml) and refluxed for 5 hours with a Dean Stark apparatus filled with molecular sieves 4A. The solution was washed, dried and evaporated in vacuo. The residue was recrystallized from ethyl acetate/toluene to give the title compound (1.79 g) as colorless prisms.

Yield: 86 %

M.P.: 182.5 - 183.5 °C

MS (m/z): 438 (M+), 409 (base peak)

IR (Nujol) $\nu_{Max}$: 3440, 1705 $cm^{-1}$

Example 12

(+)-(3R*,5S*)-(E)-N-[(R)-1-Phenylethyl]-7-[4-(4-fluorophenyl)-2,2-diethyl-2H-chromen-3-yl]-3,5-dihydroxy-6-heptenoic acid amide and (+)-(3S*,5R*)-(E)-N-[(R)-1-phenylethyl]-7-[4-(4-fluorophenyl)-2,2-diethyl-2H-chromen-3-yl]-3,5-dihydroxy-6-heptenoic acid amide:

A. (+)-(3R*,5S*)-(E)-N-[(R)-1-Phenylethyl]-7-[4-(4-fluorophenyl)-2,2-diethyl-2H-chromen-3-yl]-3,5-dihydroxy-6-heptenoic acid amide:

A solution of (±)-trans-(E)-6-[2-[2,2-diethyl-4-(4-fluorophenyl )-2H-chromen-3-yl]-1-ethenyl]-3,4,5, 6-tetrahydro-4-hydroxy-2H-pyran-2-one (24.3 g) and R-(+)-1-phenyl ethylamine (13.9 g) in toluene (110 ml) was refluxed for 3 hours. After completion of the reaction, the mixture was cooled and made acidic with 10 % hydrochloric acid, and extracted with ethyl acetate. The extract was washed, dried and evaporated in vacuo. The residue was subjected to silica-gel column chromatography [eluent: n-hexane/ethyl acetate (1:1)-]. From the fast-eluting fraction, there was obtained the title compound (13.7 g) as colorless caramels.
Yield: 41 %
$[\alpha]_D^{20}$ + 40.8° (c=0.54, chloroform)
MS (m/z): 543 ($M^+$), 105 (base peak)
IR ($CHCl_3$) $\nu_{Max}$: 3430, 1655, 1605 $cm^{-1}$

B. (+)-(3S*,5R*)-(E)-N-[(R)-1-Phenylethyl]-7-[4-(4-fluorophenyl)-2,2-diethyl-2H-chromen-3-yl]-3,5-dihydroxy-6-heptenoic acid amide:

From the slow-eluting fraction in the above (A), there was obtained the title compound (14.2 g) as colorless caramels.
Yield: 45 %
$[\alpha]_D^{20}$ + 19.5° (c=0.7 chloroform)
MS (m/z): 543 ($M^+$), 105 (base peak)
IR ($CHCl_3$) $\nu_{Max}$: 3440, 1655, 1605 $cm^{-1}$

Example 13

(+)-Trans-(E)-6-[2-(4-(4-fluorophenyl)-2,2-diethyl-2H-chromen-3-yl]-1-ethenyl]-3,4,5,6-tetrahydro-4-hydroxy-2H-pyran-2-one:

To a solution of the compound prepared in Example 12(A) (0.95 g) in ethanol (14 ml) was added a solution of sodium hydroxide (0.72 g) in water (7 ml) and the mixture was refluxed under argon atmosphere for 12 hours. After completion of the reaction, the solvent was removed by distillation. The residue was diluted with ice-water, acidified with 10 % hydrochloric acid and extracted with ethyl acetate. The extract was washed, dried and evaporated in vacuo. The residue was taken into toluene (200 ml) and refluxed for 7 hours with a Dean Stark apparatus filled with Zeolite A-4. After cooling, ethyl acetate was added and the solution was washed, dried, and evaporated to driness under reduced pressure. The residue was purified by silica-gel column chromatography [eluent: n-hexane/ethyl acetate (1:1)] and recrystallized from n-hexane/ethyl acetate to give the title compound (0.58 g) as colorless crystals.
Yield: 44 %
M.P.: 80 - 85° C
$[\alpha]_D^{20}$ = +70.2° (c=0.90, chloroform)
MS (m/z): 422 ($M^+$), 393 (base peak)
IR ($CHCl_3$) $\nu_{Max}$: 3610, 3430, 1735 $cm^{-1}$

Example 14

(-)-Trans-(E)-6-[2-[4-(4-fluorophenyl)-2,2-diethyl-2H-chromen-3-yl]-1-ethenyl]-3,4,5,6-tetrahydro-4-hydroxy-2H-pyran-2-one:

The compound prepared in Example 12(B) (0.84 g) was treated in the same manner as described in Example 13 to give the title compound (0.57 g) as colorless crystals.
Yield: 88 %
$[\alpha]_D^{20}$ -70.0° (c = 0.93, chloroform)
MS (m/z): 422 ($M^+$), 393 (base peak)
IR ($CHCl_3$) $\nu_{Max}$: 3610, 3430, 1735 $cm^{-1}$

Example 15

Sodium (+)-(3R*,5S*)-(E)-7-[-4-(4-fluorophenyl)-2,2-diethyl-2H-chromen-3-yl]-3,5-dihydroxy-6-heptenoate:

To a stirred and ice-cooled solution of the compound prepared in Example 13 (0.52 g) in methanol (5 ml) was added an aqueous sodium hydroxide solution (2N, 1.0 ml) and the mixture was stirred at room temperature for 0.5 hour. After evaporation of the solvent, the residue was purified by column chromatography [eluent: methanol/water (9:1)] using a non-ionic absorption resin (trade name: Daiyaion HP-20 manufactured by Mitsubishi Kasei K.K.) to give the title compound (0.52 g) as colorless powder.
Yield: 88 %
$[\alpha]_D^{20}$ +59.7° (c = 0.7, methanol)
MS (FAB) (m/z): 485 ($M^+$ + Na), 463 ($M^+$ + 1)
IR (Nujol) $\nu_{Max}$: 3320, 1570 $cm^{-1}$

Example 16

Sodium (-)-(3R*,5S*)-(E)-7-[4-(4-fluorophenyl)2,2-diethyl-2H-chromen-3-yl]-3,5-dihydroxy-6-heptenoate:

(-)-Trans-(E)-6-[2-[4-(4-fluorophenyl)-2,2-diethyl- 2H-chromen3-yl]-1-ethenyl]-3,4,5,6-tetrahydro-4-hydroxy-2H-pyran-2-one (0.52 g) was treated in the same manner as described in Example 15 to give the title compound (0.52 g) as colorless powder.
Yield: 88 %
$[\alpha]_D^{20}$ -59.0° (c = 0.89, methanol).
The product had the same IR and MS values as those of the compound prepared in Example 15.

Example 17

(+)-(3R*,5S*)-(E)-N-[(R)-1-Phenylethyl]-7-[4-(4-fluorophenyl)-2,2-diethyl-2H-thiochromen-3-yl]-3,5-dihydroxy-6-heptenoic acid amide and (+)-(3S*,5R*)-(E)-N-[(R)-1-phenylethyl]-7-[4-(4-fluorophenyl)-2,2-diethyl-2H-thiochromen-3-yl]-3,5-dihydroxy-6-heptenoic acid amide:

A. (+)-(3R*,5S*)-(E)-N-[(R)-1-Phenylethyl]-7-[4-(4-fluorophenyl)-2,2-diethyl-2H-thiochromen-3-yl]-3,5-dihydroxy-6-heptenoic acid amide:

A solution of (±)-trans-(E)-6-[2-[-2,2-diethyl-4-(4-fluorophenyl)-2H-thiochromen-3-yl]-1-ethenyl]-3,4,5,6-tetrahydro-4-hydroxy-2H-pyran-2-one (324 mg) and R-(+)-1-phenylethylamine (269 mg) in toluene (8 ml) was refluxed for 3 hours. After completion of the reaction, the mixture was cooled, acidified with 10 % hydrochloric acid and extracted with ethyl acetate. The extract was washed, dried and evaporated in vacuo. The residue was subjected to silicagel column chromatography [eluent: n-hexane/ethyl acetate (1:1)]. From the fast-eluting fraction, there was obtained the title compound (180 mg) as colorless caramel.
Yield: 44 %
$[\alpha]_D^{20}$ + 40.3° (c = 1.4, chloroform)
MS (FAB) (m/z): 560 ($M^+$ + 1), 106 (base peak)
IR ($CHCl_3$) $\nu_{Max}$: 3280, 1640 $cm^{-1}$

B. (+)-(3S*,5R*)-(E)-N-[(R)-1-Phenylethyl]-7-[4-(4-fluorophenyl)-2,2-diethyl-2H-thiochromen-3-yl]-3,5-dihydroxy-6-heptenoic acid amide:

From the slow-eluting fraction, there was obtained the title compound (150 mg) as colorless caramel.
Yield: 37 %
$[\alpha]_D^{20}$ + 30.3° (c=1.0, chloroform)
MS (FAB) (m/z): 560 ($M^+$ +1), 106 (base peak)
IR ($CHCl_3$) $\nu_{Max}$: 3280, 1640 $cm^{-1}$

Example 18

(+)-Trans-(E)-6-[2-[4-(4-fluorophenyl)-2,2-diethyl-2H-thiochromen-3-yl]-1-ethenyl]-3,4,5,6-tetrahydro-4-hydroxy-2H-pyran-2-one:

To a solution of the compound prepared in Example 17(A) (137 mg) in ethanol (3 ml) was added a solution of sodium hydroxide (108 mg) in water (1.5 ml) and the mixture was refluxed under argon atmosphere for 12 hours. After completion of the reaction, the solvent was removed by distillation. The residue was diluted with ice-water, acidified with 10 % hydrochloric acid and extracted with ethyl acetate. The extract was washed, dried and evaporated in vacuo. The residue was taken into toluene (5 ml) and the mixture was refluxed for 7 hours with a Dean Stark apparatus filled with Zeolite A-4. After cooling, ethyl acetate was added and the mixture was washed, dried and evaporated to driness under reduced pressure. The residue was purified by silica-gel column chromatography [eluent: n-hexane/ethyl acetate (1:1)] and recrystallized from n-hexane/ethyl acetate to give the title compound (68 mg) as colorless crystals.
Yield: 63 %
M.P.: 124 - 126° C
$[\alpha]_D^{20}$ = + 70.5° (c=1.4, chloroform)
MS (m/z): 438 ($M^+$), 409 (base peak)
IR (Nujol) $\nu_{Max}$: 3440, 1720 $cm^{-1}$

Example 19

(-)-Trans-(E)-6-[2-[4-(4-fluorophenyl)-2,2-diethyl-2H-thiochromen-3-yl]-1-ethenyl]-3,4,5,6-tetrahydro-4-hydroxy-2H-pyran-2-one:

The compound prepared in Example 17(B) (102 mg) was treated in the same manner as described in Example 18 to give the title compound (64 mg) as colorless crystals.
Yield: 81 %
M.p.: 124 - 126° C
$[\alpha]_D^{20}$ - 69.9° (c=1.2, chloroform)
MS (m/z): 438 ($M^+$), 409 (base peak)
IR (Nujol) $\nu_{Max}$: 3420, 1720 $cm^{-1}$

Example 20

Sodium (+)-(3R*,5S*)-(E)-7-[4-(4-fluorophenyl)-2,2-diethyl-2H-thiochromen-3-yl]-3,5-dihydroxy-6-heptenoate:

To a stirred and ice-cooled solution of the compound prepared in Example 18 (64 mg) in methanol (2 ml) was added an aqueous sodium hydroxide solution (1N, 0.6 ml) and the mixture was stirred for 0.5 hour. After evaporation at room temperature, the residue was purified by column chromatography [eluent: methanol/water (9:1)] using a nonionic absorption resin (trade name: Daiyaion HP-20 manufactured by Mitsubishi Kasei K.K.) to give the title compound (66 mg) as colorless powder.
Yield: 95 %

$[\alpha]_D^{20}$ + 68.2° (c = 1.4, methanol)
MS (FAB) (m/z): 501 ($M^+$ + Na)
IR (Nujol) $\nu_{Max}$: 3320, 1580 $cm^{-1}$

Example 21

Sodium (-)-(3R*,5S*)-(E)-7-[4-(4-fluorophenyl)-2,2-diethyl-2H-thiochromen-3-yl]-3,5-dihydroxy-6-heptenoate:

(-)-Trans-(E)-6-[2-[4-(4-fluorophenyl)-2,2-diethyl-2H-thiochromen-3-yl]-1-ethenyl]-3,4,5,6-tetrahydro-4-hydroxy-2H-pyran-2-one (60 mg) was treated in the same manner as described in Example 20 to give the title compound (67 mg) as colorless powder.
$[\alpha]_D^{20}$ - 65.1° (c = 1.3, methanol)
The product had the same IR and MS values as those of the compound prepared in Example 20.

[Preparation of starting compounds]

Reference Example 1

4-(4-Fluorophenyl)-2,2-dimethyl-2H-chromene:

A. 4-(4-Fluorophenyl)-2,2-dimethylchroman-4-ol:

To a solution of p-fluorobromobenzene (12.76 g) in ether in a dry ice-acetone bath was dropwise added a solution of n-butyllithium in n-hexane (1.6 M, 43.4 ml). The mixture was stirred for 15 minutes during which the temperature was allowed to raise. Then the mixture was again cooled and there was dropwise added a solution of 2,2-dimethyl-chroman-4-one (6.12 g). After 1 hour, the temperature was raised to -10° C and the reaction mixture was poured into a mixture of an aqueous ammonium chloride solution and ice. To the mixture was added ethyl acetate and the organic phase was separated, washed, dried and evaporated. The residue was purified by silica-gel column chromatography and recrystallized from n-hexane to give the title compound (2.7 g) as colorless prisms.
M.P.: 93 - 94° C

B. 4-(4-fluorophenyl)-2,2-dimethyl-2H-chromene:

A mixture of the compound A (0.695 g) in toluene was refluxed in the presence of p-toluenesulfonic acid (78 mg) for 1 hour. After cooling, the solvent was removed by distillation to give the title compound (0.65 g) as colorless oil.
IR (Neat): 1635, 1603 $cm^{-1}$

Reference Example 2

4-( 4-Fluorophenyl)-2,2-diethyl-2H-chromene:

A. 4′-Fluoro-2-hydroxybenzophenone:

A mixture of salicylic acid (10.0 g), aluminum chloride (0.02 g) and thionyl chloride (10 ml) was stirred at 40 to 50° C for 2 hours. Excess thionyl chloride was removed by distillation and the residue was dissolved in fluorobenzene. To the cooled solution was added portionwise aluminum chloride (15.6 g) and

the mixture was stirred at 60°C for 12 hours. The reaction mixture was poured into a mixture of ice and hydrochloric acid, and extracted with ethyl acetate. The extract was washed, dried and evaporated in vacuo. The residue was treated with charcoal and then recrystallized from n-hexane to give the title compound (7.12 g) as yellow prisms.
M.P.: 69 - 71.5°C

B. 4-(4-Fluorophenyl)-2H-chromen-2-one:

A reaction vessel was charged with a mixture of the compound A (12.958 g), ethyl (triphenylphosphoranilidene)-acetate (20.88 g) and toluene and purged with argon and then sealed. After stirring at 170 to 180°C for 65 hours, the solvent was removed by distillation. The residue was purified by silica-gel flash column chromatography and then recrystallized from ethyl acetate/isopropyl ether to give the title compound (10.64 g) as pale yellow needles.
M.P.: 156 - 157°C

C. 2-[3-Ethyl-1-(4-fluorophenyl)-3-hydroxy-1-pentenyl]phenol:

To a mixture of magnesium (3.38 g) and ether was added a solution of ethyl bromide (13.79 g) in ether under refluxing over 15 minutes and the mixture was refluxed for another 15 minutes. To the ice-cooled mixture was dropwise added a solution of the compound B (10.13 g) in tetrahydrofuran and the mixture was stirred for 30 minutes and then allowed to warm to room temperature over 15 minutes. The reaction mixture was poured into a mixture of ice and aqueous ammonium chloride solution and extracted with ethyl acetate. The extract was washed, dried and evaporated. The residue was recrystallized from n-hexane/ethyl acetate to give the title compound (9.85 g) as colorless prisms.
M.P.: 123 - 124.5°C

D. 4-(4-Fluorophenyl)-2,2-diethyl-2H-chromene:

A solution of the compound C (9.4 g) in tetrahydrofuran was added dropwise to conc. hydrochloric acid and the mixture was stirred for 1 hour. The reaction mixture was poured onto ice and extracted with ethyl acetate. After treatment with charcoal, the extract was evaporated in vacuo to give the title compound (8.7 g) as pale red oil.
IR (Neat): 1600 $cm^{-1}$

Reference Example 3

A. 3-Ethyl-3-phenylthiopentanoic acid:

To a stirred and ice-cooled mixture of thiophenol (11.52 g) and 3-ethyl-2-pentenoic acid (13.41 g) was added methanesulfonic acid (13.6 ml) and the whole was stirred at room temperature overnight. To the mixture were added ether and water. The ethereal layer was separated and the aqueous layer was extracted with ether. The combined ethereal layer was washed with water, dried over magnesium sulfate, and the solvent was removed by distillation. The residual oil was crystallized from n-hexane to give the title compound (17.24 g).
Yield: 68.6 %
M.P.: 77 - 78°C

B. 2,2-Diethyl-3,4-dihydro-1H-thiochromen-4-one:

To polyphosphoric acid (83 g) was added the compound A (17.24 g) and the mixture was stirred in argon atmosphere at 50 to 70°C for 6 hours. The reaction mixture was cooled and the polyphosphoric acid was decomposed with water, followed by extraction of the product with ether. The extract was washed with

water, 10% sodium hydroxide and brine successively, and dried. The solvent was removed by distillation to give oil (14.6 g), which was purified by silica-gel column chromatography [eluent: n-hexane/ethyl acetate (40:1)] to give the title compound (11.27 g) as yellow oil.
Yield: 71 %
MS (m/z): 220 ($M^+$), 191 (base peak)
IR: 1680 $cm^{-1}$ (C=O)

C. 2,2-Diethyl-4-(4-fluorophenyl)-2H-thiochromene

A solution of para-fluorobromobenzene (8.82 g) in ether (90 ml) was cooled to -60°C under argon atmosphere. There was added dropwise a solution of n-butyllithium in hexane (1.6 M, 29 ml), and the mixture was stirred at -25°C for 30 minutes. The solution of para-fluorophenyllithium thus prepared was again cooled to -60°C and thereto was dropwise added a solution of the compound B (9.26 g) in ether (85 ml). After 1 hour, the reaction was quenched with a saturated aqueous ammonium chloride solution and the organic layer was separated. The organic layer was washed with water, dried and evaporated in vacuo. The residual oil was heated at 100°C in toluene (50 ml) in the presence of catalytic amount of para-toluenesulofonic acid for 30 minutes. After cooling, the solution was washed with water and dried and the solvent was removed by distillation. The residue was purified by silica-gel column chromatography [eluent: hexane/ethyl acetate (200:1)] to give the title compound (4.42 g) as oil.
Yield: 35.2 %
MS (m/z): 298 ($M^+$), 269
NMR ($CDCl_3$) (90 MHz) $\delta$ : 0.97 (6H, t, J = 7 Hz; $CH_2C\underline{H}_3$ x 2), 5.66 (1H, s; =$C\underline{H}$ -)

D. 3-Bromo-2,2-diethyl-4-(4-fluorophenyl)-2H-thiochromene:

A solution of the compound C (1.5 g) in chloroform was cooled in an ice bath and thereto was dropwise added a solution of bromine (0.9 g) in chloroform (5 ml). After stirring for 1 hour, the solution was washed with water, an aqueous saturated sodium hydrogen carbonate solution and water successively, and dried. The solvent was removed by distillation to give the title compound (1.8 g) as oil. Yield: 95 %
MS (m/z): 378 ($M^+$), 349 (base peak)

E. (E)-3[2,2-Diethyl-4-(4-fluorophenyl)-2H-thiochromen-3-yl]-2-propen-1-ol:

A mixture of the compound D (0.55 g), (E)-3-tributylstannyl-2-propen-1-ol (0.76 g) and bistriphenylphosphine palladium chloride (0.05 g) in dioxane (5 ml) was refluxed under argon atmosphere for 6 hours. After cooling, an aqueous solution of potassium fluoride was added to the mixture and the precipitated insoluble materials were filtered through Celite pad. The product was extracted with ethyl acetate and the extract was washed with water, dried and evaporated in vacuo. The residue was purified by silica-gel column chromatography [eluent: n-hexane/ethyl acetate (4:1)] to give the title compound (0.38 g) as oil.
Yield: 73 %
IR (Neat): 3360 $cm^{-1}$ (OH)
MS (m/z): 354 ($M^+$), 325 (base peak)

F. (E)-3-[2,2-Diethyl-4-(4-fluorophenyl)-2H-thiochromen-3-yl]-2-propenal:

A mixture of the compound E (0.34 g) and active manganese dioxide (0.8 g) in methylene chloride (3 ml) was refluxed for 3 hours. After completion of the reaction, the insoluble materials were removed by filtration and washed with methylene chloride. The combined filtrate and the washings were evaporated under reduced pressure. The residue was purified by silica-gel column chromatography [eluent: n-hexane/ethyl acetate (10:1)] to give the title compound (0.28g) as crystals.
Yield: 80 %
M.P.: 58 - 59°C

## Claims

1. A chromene or thiochromene derivative of the formula:

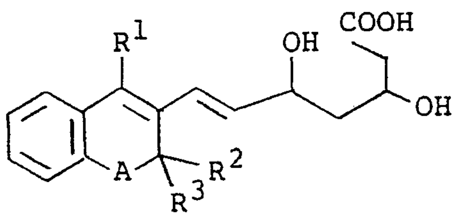

(I)

wherein A is oxygen or sulfur atom, $R^1$ is a halogenophenyl, $R^2$ and $R^3$ are the same or different and are each a lower alkyl or $R^2$ and $R^3$ are combined together to form $-(CH_2)_n-$ in which n is an integer of 4 to 6, or a pharmaceutically acceptable ester, amide, lactone or salt thereof.

2. The compound according to claim 1 which has the formula:

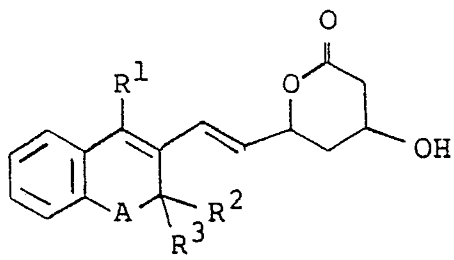

wherein A, $R^1$, $R^2$ and $R^3$ are the same as defined in claim 1.

3. The compound according to claim 1 or 2 wherein A is oxygen atom, and $R^2$ and $R^3$ are the same or different and are each an alkyl group having 1 to 4 carbon atoms.

4. The compound according to claim 1 or 2 wherein A is sulfur atom and $R^2$ and $R^3$ are ethyl.

5. The compound according to claim 1 wherein $R^2$ and $R^3$ are ethyl.

6. The compound according to any one of claims 3 to 5 wherein $R^1$ is fluorophenyl.

7. The compound according to any one of claims 3 to 5 wherein $R^1$ is 4-fluorophenyl.

8. The compound according to claim 7 which is trans-(E)-6-[ 2-[4-(4-fluorophenyl)-2, 2-diethyl-2H-chromen-3-yl]-1-ethenyl]-3,4,5,6-tetrahydro-4-hydroxy-2H-pyran-2-one.

9. The compound according to claim 7 which is trans-(E)-6-[2-[4-(4-fluorophenyl)-2,2-diethyl-2H-thiochromen-3-yl]-1-ethenyl]-3,4,5,6-tetrahydro-4-hydroxy-2H-pyran-2-one.

10. A process for preparing a chromene or thiochromene derivative of the formula:

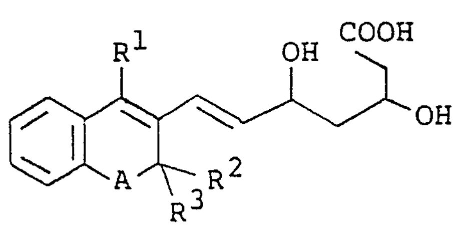

(I)

wherein A is oxygen or sulfur atom, $R^1$ is a halogenophenyl, $R^2$ and $R^3$ are the same or different and are each a lower alkyl or $R^2$ and $R^3$ are combined together to form $-(CH_2)_n-$ in which n is an integer of 4 to 6, or a pharmaceutically acceptable ester, amide, lactone or salt thereof, which comprises reducing a compound of the formula:

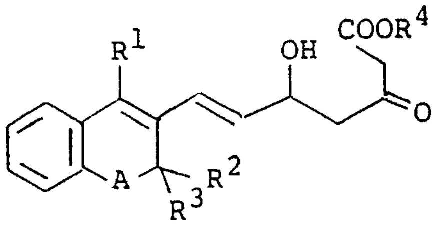

(IV)

wherein A, $R^1$, $R^2$ and $R^3$ are as defined above, $-COOR^4$ is a carboxyl group which may have a protecting group, removing the protecting group when $-COOR^4$ is a carboxyl group having a protecting group, and optionally subjecting the product to esterification, amidation or lactonation reaction or forming a salt of the product.

11. A process for preparing a chromene or thiochromene derivative of the formula:

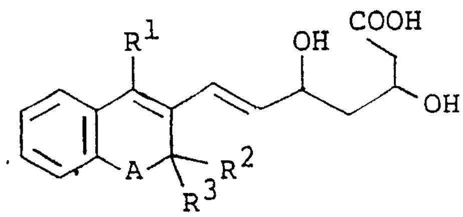

(I)

wherein A is oxygen or sulfur atom, $R^1$ is a halogenophenyl, $R^2$ and $R^3$ are the same or different and are each a lower alkyl or $R^2$ and $R^3$ are combined together to form $(CH_2)_n$- in which n is an integer of 4 to 6, or a pharmaceutically acceptable ester, amide, lactone or salt thereof, which comprises reacting an aldehyde compound of the formula:

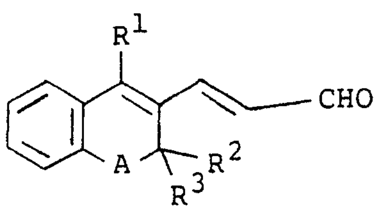

(II)

wherein A, $R^1$, $R^2$ and $R^3$ are as defined above, or a salt thereof, with an acetoacetic acid compound of the formula:

$CH_3COCH_2COOR^4$ (III)

wherein $-COOR^4$ is a carboxyl group which may have a protecting group, or a salt thereof, to give a compound of the formula:

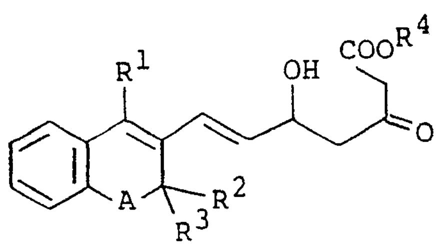

wherein A, $R^1$, $R^2$, $R^3$ and $-COOR^4$ are as defined above, or a salt thereof, reducing the product, and removing a protecting group when $-COOR^4$ is carboxyl group having the protecting group, and optionally subjecting the product to esterification, amidation or lactonation reaction or forming a salt of the product.

12. An aldehyde compound of the formula:

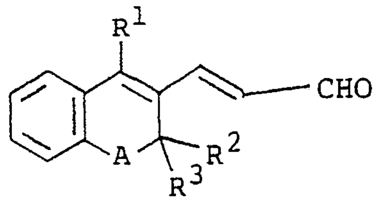

(IV)

wherein A is oxygen or sulfur atom, $R^1$ is a halogenophenyl, $R^2$ and $R^3$ are the same or different and are each a lower alkyl or $R^2$ and $R^3$ are combined together to form $-(CH_2)_n-$ in which n is an integer of 4 to 6, or a salt thereof.

13. A pharmaceutical composition which comprises an effective amount of the chromene or thiochromene derivative as set forth in claim 1 as an active ingredient in admixture with a pharmaceutically acceptable carrier or diluent.

14. Use of a chromene or thiochromene derivative as claimed in anyone of the claims 1 to 9 for preparing a pharmaceutical composition for prophylaxis and treatment of hyperlipidemia, coronary disease, arteriosclerosis, familial hypercholesterolemia and xanthoma.

Claims for the following Contracting States: ES, GR

1. A process for preparing a chromene or thiochromene derivative of the formula:

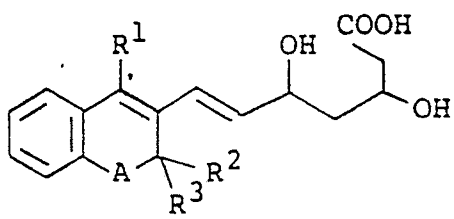

(I)

wherein A is oxygen or sulfur atom, $R^1$ is a halogenophenyl, $R^2$ and $R^3$ are the same or different and are each a lower alkyl or $R^2$ and $R^3$ are combined together to form $-(CH_2)_n-$ in which n is an integer of 4 to 6, or a pharmaceutically acceptable ester, amide, lactone or salt thereof, which comprises reducing a compound of the formula:

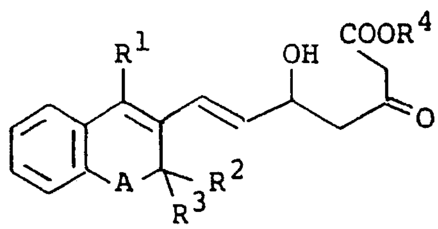

(IV)

wherein A, $R^1$, $R^2$ and $R^3$ are as defined above, $-COOR^4$ is a carboxyl group which may have a protecting group, removing the protecting group when $-C00R^4$ is a carboxyl group having a protecting group, and optionally subjecting the product to esterification, amidation or lactonation reaction or forming a salt of the product.

2. A process for preparing a chromene or thiochromene derivative of the formula:

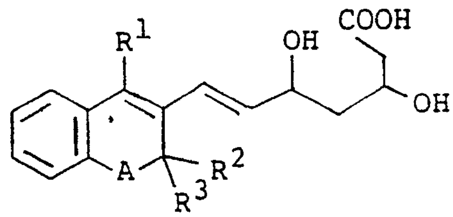

(I)

wherein A is oxygen or sulfur atom, R[1] is a halogenophenyl, $R^2$ and $R^3$ are the same or different and are each a lower alkyl or $R^2$ and $R^3$ are combined together to form $-(CH_2)_n-$ in which n is an integer of 4 to 6, or a pharmaceutically acceptable ester, amide, lactone or salt thereof, which comprises reacting an aldehyde compound of the formula:

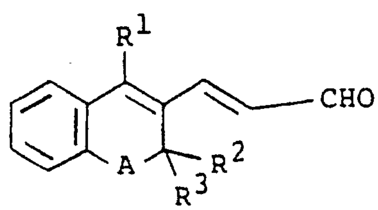

(II)

wherein A, $R^1$, $R^2$ and $R^3$ are as defined above, or a salt thereof, with an acetoacetic acid compound of the formula:

$CH_3COCH_2COOR^4$ (III)

wherein $-COOR^4$ is a carboxyl group which may have a protecting group, or a salt thereof, to give a compound of the formula:

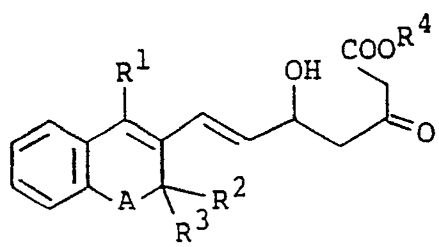

wherein A, $R^1$, $R^2$, $R^3$ and $-COOR^4$ are as defined above, or a salt thereof, reducing the product, and removing a protecting group when $-COOR^4$ is carboxyl group having the protecting group, and optionally subjecting the product to esterification, amidation or lactonation reaction or forming a salt of the product.

3. The use of a chromene or thiochromene derivative having the formula (I)

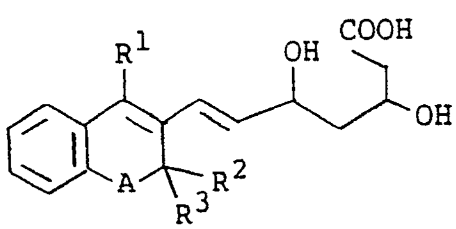

(I)

wherein A, $R^1$, $R^2$ and $R^3$ have the meanings as stated above, or a pharmaceutically acceptable ester, amide, lacton or salt thereof, for the preparation of a pharmaceutical composition for prophylaxis and treatment of hyperlipidemia, coronary disease, arteriosclerosis, familial hypercholesterolemia and xanthoma.